# EUROPEAN PATENT APPLICATION

(11) **EP 4 218 957 A1**
(43) Date of publication of application: **02.08.2023**
(21) Application number: 22386003.2
(22) Date of filing: 27.01.2022
(51) Int. Cl.: A63B 21/00, A63B 21/055, A63B 21/068, A63B 23/12, A63B 71/06, A61B 5/103, A61B 5/11, A61B 5/22, A61B 5/00, A63B 21/072, A63B 24/00, G01L 5/00, G01L 1/20

(54) **SMART EXERCISE HANDLE**

(71) Applicant: Train Me Tech Ltd, London W1U 7ND (GB)
(72) Inventor: KYVRANOGLOU, Anestis, Ioannina 45500 (GR); STAMATOPOULOS, Petros, Athens 15452 (GR); TZAMOS, Nikolaos, Halandri 15231 (GR); DIDASKALOU, Alexandros, Attili 15343 (GR)
(74) Representative: Boult Wade Tennant LLP

(57) **Abstract**

A smart exercise handle for bodyweight or resistance exercise is provided. The handle comprises: a hand-grip for a user to grip; and a ground-engaging section extending from the hand-grip. The ground-engaging section is for contacting the ground to support the smart exercise handle during exercise. The handle further comprises: at least one force sensor arranged on the hand-grip to detect a force applied to the hand-grip and generate a signal indicative thereof. The handle further comprises: a processor configured to receive the signal and generate information related to the force applied to the hand-grip.

## Description

### Background

The present disclosure relates to a smart exercise handle, particularly for use in home exercise. This can be known, for example, as smart grips. Of course, the smart exercise handle may be used in gym or outdoor settings as well.

At home exercise is an attractive option for many people, and there are a large number of devices which can be used in this environment. Traditionally, at home exercise consisted of weights, resistance exercises and the like. Gradually, home versions of popular gym equipment such as treadmills, exercise bikes and rowing machines were developed. Further recent developments have incorporated so-called "smart" devices into home workouts. Generally a smart device is one which is capable of some independent action such as sensing and recording exercises carried out thereon.

Examples of such smart home equipment includes Peloton exercise bikes, Tonal strength machine, Mirror exercise machine, Tempo home gym, JAXJOX interactive studio, Ergatta rower, and the like. These pieces of equipment are quite large and bulky, and take up significant parts of the room. For people without a dedicated home gym this can be a problem. These pieces of equipment are also not easily portable and hence cannot be easily used where desired.

Push-up bars including sensors for detecting a force through are bar are known such as the Sportstech PBX300. Each handle consists of two feet, with a bent bar joining them together. At the centre of the bent bar is a simple hand-grip. Since the device is solely used for push-ups, the force is either measured at the interface between the bent bar and the feet, or at the feet themselves effectively in the form of a scale. This is limited to push-ups and does not allow use for a variety of exercise types.

US 2019/0175973 A1 discloses a piece of fitness equipment including two handles each including a gripping part gripped by hands and an elastic member-coupling part to which an end portion of an elastic member configured to extend between the two handles is coupled; and a supporting base provided with a supporting surface provided at a location opposite the gripping part of the handle, and supported on a bottom surface, wherein the handle and supporting base assemblies are used to perform push-up exercises, and the elastic member is coupled between the two handles to perform chest expander exercises.

There is therefore a need for an improved exercise device.

### Summary

A smart exercise handle for bodyweight or resistance exercise is provided according to claim 1. This smart exercise handle can be used to carry out an interactive exercise session while being easily portable and suitable for a wide variety of exercises. The term "smart" simply means that the device carries out some independent action - in this case at least force sensing. The smart exercise handle can simply be referred to as an exercise handle without any change in characteristics.

The at least one force sensor may be formed as a force sensitive film wrapped around at least a portion of the hand-grip. Wrapping the force sensitive film around at least a portion of the hand-grip helps to ensure forces applied to the hand-grip are sensed. Particularly, forces applied to the portion of the hand-grip which is covered by the sensor are sensed.

The force sensitive film may be wrapped around a majority of the circumference the hand-grip. This helps to avoid forces applied to the hand-grip being inadvertently not recorded.

The smart exercise handle may further comprise an elastomeric padding layer covering the force sensitive film. Many force sensitive films or resistors are too sensitive and the range of force that can be measured is up to around 10 kilograms. The padding absorbs some of the force applied to smart exercise handle and thereby increases this range.

The elastomeric padding layer may be silicon. This is a particularly effective material for the padding layer.

The force sensitive film may be separated into a plurality of sensing areas, each sensing area arranged to detect a force applied to the hand-grip in a different direction. The detection of forces in different directions allows for further detail about exercise carried out with the smart exercise handle to be determined.

Each sensing area may be arranged to detect a force applied to the hand-grip in a reference direction for that sensing area, and there may be a corresponding sensing area arranged to detect a force applied to the hand-grip in an opposite direction to the reference direction, wherein the processor is configured to compare the signal from each sensing area to the signal from the corresponding sensing area. Having sensing areas essentially on opposite sides of the hand-grip means that the forces can be summed to determine the net force acting in a direction. This can help determine the type of exercise being carried out, and differentiate from someone squeezing the hand grip. As will be appreciated, the sensing areas are not points and hence the force detected may not be perfectly opposite.

The at least one force sensor may comprise a plurality of force sensitive resistors separated into a plurality of sensing areas, each sensing area comprising at least one force sensitive resistor of the plurality of force sensitive resistors and arranged to detect a force applied to the hand-grip in a different direction. The detection of forces in different directions allows for further detail about exercise carried out with the smart exercise handle to be determined.

The at least one force sensitive resistor in each sensing area may be arranged to detect a force applied to the hand-grip in a reference direction for that sensing area, and the at least one force sensitive resistor in a corresponding sensing area may be arranged to detect a force applied to the hand-grip in an opposite direction to the reference direction, wherein the processor is configured to compare the signal from each sensing area to the signal from the corresponding sensing area. Having sensing areas essentially on opposite sides of the hand-grip means that the forces can be summed to determine the net force acting in a direction. This can help determine the type of exercise being carried out, and differentiate from someone squeezing the hand-grip. As will be appreciated, the sensing areas are not points and hence the force detected may not be perfectly opposite.

The plurality of sensing areas may be four sensing areas. Four sensing areas allows the hand-grip to be separated into quadrants. This can give a good measure of the forces applied thereto.

The hand-grip may be generally circular in cross-section and each sensing area and its corresponding sensing area may be diametrically opposed in the cross-section. Circular hand-grips are convenient for gripping and diametrically opposed sensing areas improves the ease of the forces being summed to determine the net force acting in a direction. This can help determine the type of exercise being carried out, and differentiate from someone squeezing the hand-grip

The smart exercise handle may further comprise, connected to the processor, one or more of: an accelerometer; a gyroscope; and/or a heart-rate sensor. That is, the smart exercise handle may have any of these further sensing components. Any combination of two or more of these further sensing components may also be used. These further sensors can be used to determine more information about the user and/or the exercise being carried out. This can be used to help determine the type of exercise. These additional sensors can also help in determining the "form" of a user of the smart exercise handle - whether they are carrying out an intended exercise correctly.

The smart exercise handle may further comprise a vibration generator. A vibration generator allows the smart exercise handle to provide feedback to a user.

The smart exercise handle may further comprise a transmitter arranged to transmit the signal indicative of a force applied to the hand-grip and/or the information related to the force applied to the hand-grip to a remote device. The remote device may be a mobile phone, such as a smart phone. Transmitting this information to a remote device means that the processing of any data can be carried out remotely on a device with greater processing power than the smart exercise handle. This is also a convenient way for a user to track and record their workouts.

The ground-engaging section may comprise a first ground-engaging protrusion and a second ground-engaging protrusion, with the hand-grip extending therebetween. This can provide a stable base for the smart exercise handle for use in exercises such as push-ups.

The first ground-engaging protrusion may be angled with respect to the second ground-engaging protrusion such that first ground-engaging protrusion and the second ground-engaging protrusion are outwardly-splayed. Outwardly-splayed protrusions can improve the stability of the smart exercise handle for use in exercises such as push-ups.

The first ground-engaging protrusion and the second ground-engaging protrusion may each have a width greater than the width of the hand-grip. This greater width can improve the stability of the smart exercise handle for use in exercises such as push-ups.

The smart exercise handle may further comprise an attachment section for removably attaching a resistance band to the smart exercise handle. Attaching a resistance band to the smart exercise handle greatly increases the range of exercises which can be carried out. With the resistance band attached the at least one force sensor is still arranged to detect a force applied to the hand-grip and hence still operates to generate a signal indicative of this force to provide information about a workout using the resistance band.

The attachment section may comprise a first attachment point and a second attachment point, with the hand-grip extending therebetween. This can help distribute the resistive force of the resistance band across the smart exercise handle.

The hand-grip may define a central longitudinal axis extending along the hand-grip, and the first attachment point and the second attachment point may be positioned on the central longitudinal axis. Having the resistance band attached along this central longitudinal axis means that the band is centrally positioned and forces are therefore more evenly distributed.

The attachment section may be configured to allow free rotation of the resistance band. This means that during an exercise using the resistance band the band is able to rotate as required. This ensures even distribution of the forces generated and prevents an undesired twisting force on the smart exercise handle.

A kit is provided according to claim 12, which includes at least one smart exercise handle. This kit includes a smart exercise handle and a resistance band. Of course, the kit may have a plurality of resistance bands, with different elastic modulus. Multiple types of exercise can be carried out with this kit and the smart exercise handle is used to sense information relating to this exercise. Particularly this kit can carry out both bodyweight and resistance exercises.

The first attachment mechanism and the second attachment mechanism may each comprise a ball lock pin for engaging with the attachment sections of the first exercise handle and the second exercise handle. A ball lock pin mechanism is an effective mechanism to attach the resistance band.

The first resistance band may further comprise a third attachment mechanism at the first end and a fourth attachment mechanism at the second opposite end. Having multiple attachment mechanisms at each end allows the first resistance band to be attached to each smart exercise handle at multiple points - such as at opposing ends of the smart exercise handle.

The first resistance band may comprise a central segment which splits into two strands at the first and the second end, with each strand having an attachment mechanism. This is an effective way to provide a resistance band with multiple attachment points at either end.

The kit may include a second smart exercise handle. Having two smart exercise handles means that each handle used during an exercise session is able to independently detect force data. This also allows for an equal weight and standing base for use of the smart exercise handle.

A further kit is provided according to claim 13, which includes at least one smart exercise handle. This kit includes smart exercise handles which do not necessarily have an attachment mechanism. It is therefore directed towards a kit primarily for bodyweight exercises and the ability to detect forces generated during such exercises. Of course, any kit may have an attachment mechanism for resistance bands and resistance workouts.

A method of operating a smart exercise handle is provided according to claim 14. This method detects forces during an exercise and indicates the information to a user so that they are provided with information about the exercise. This may be, for example, via a vibration generator or LED on the smart exercise device, and/or via a remote device such as a mobile phone or smart phone.

A further method of operating a smart exercise handle is provided according to claim 15. This method uses a smart exercise handle with force sensing areas arranged to sense forces in opposite directions. In further examples, the directions may not be directly opposite but may instead simply be offset from one another. The method uses the sensed forces from these opposite directions to determine a type of exercise being carried out.

Particularly, this may be useful for differentiating between a force applied during a workout and a squeezing of the smart exercise handle. In examples including one or more of: an accelerometer; a gyroscope; and/or a heart-rate sensor, these components may further be used in the method to determine the type of exercise being carried out.

### Brief Description of the Drawings

The present specification will make reference to the accompanying drawings in which:
Figure 1 shows a perspective view of a smart exercise handle;
Figure 2 shows a side view of the smart exercise handle of Figure 1;
Figure 3 shows a top view of the smart exercise handle of Figure 1;
Figure 5 shows a side view of the smart exercise handle of Figure 1 being used for exercise;
Figure 6 shows a perspective view of a resistance band being attached to the smart exercise handle of Figure 1;
Figure 6A shows an expanded perspective view of a resistance band being attached to the smart exercise handle of Figure 1;
Figure 7 shows a perspective view of the smart exercise handle of Figure 1 with a resistance band attached;
Figure 8 shows a side view of the smart exercise handle and resistance band of Figure 7 being used for exercise; and
Figure 9 shows a schematic view of a force sensitive film which may be used in a smart exercise handle.

### Detailed Description of the Drawings

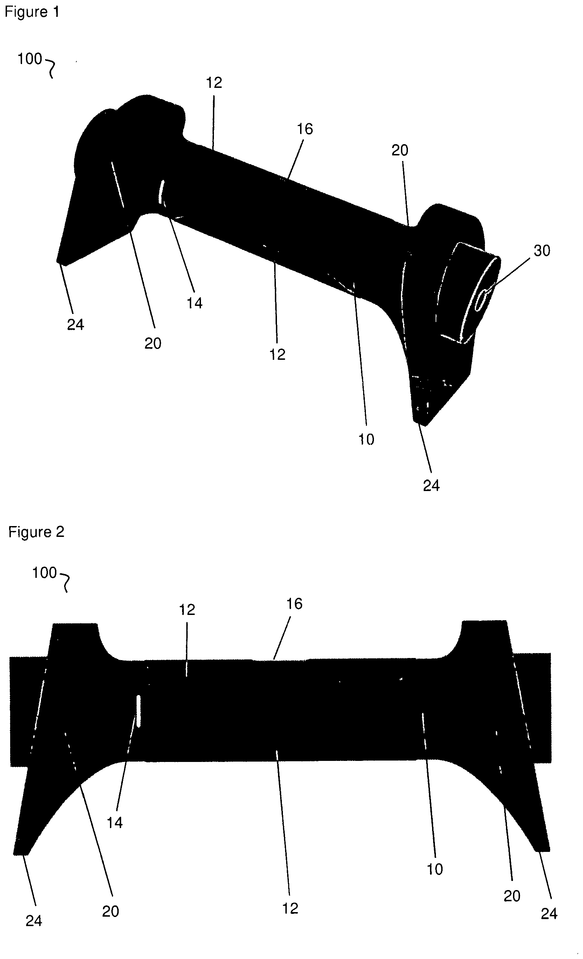

Figures 1 to 3 show a smart exercise handle 100 according to the present disclosure. As noted above, the term "smart" simply means that the device carries out some independent action - in this case at least force sensing. The smart exercise handle 100 can simply be referred to as an exercise handle 100 without any change in characteristics. Any reference to an exercise handle 100 or smart exercise handle 100 below must therefore be considered interchangeable.

The smart exercise handle 100 can be used for bodyweight and/or resistance exercise. Bodyweight exercises are strength-training exercises that use an individual's own weight to provide resistance against gravity. Bodyweight training can use simple movements like pushing, pulling, squatting, bending, twisting and balancing. This may include, for example, push-ups, jumping, boxing, and/or yoga. Resistance exercise refers to movement of muscles against a resistive force, such as that provided by a resistance band.

The smart exercise handle 100 includes a hand-grip 10 for a user to grip. This hand-grip 10 may be generally elongate. That is, it may be an elongate hand-grip 10. The hand-grip may define a longitudinal axis, which may be along its greatest dimension. This longitudinal axis may extend generally axially along the hand-grip 10. The hand-grip 10 may be generally shaped and sized for an adult hand. Of course, there may be multiple smart exercise handles 100 with different grip sizes, such as for children and/or different hand sizes. The hand-grip 10 may be generally circular in cross-section. That is, the hand-grip 10 may be formed as a cylinder. The hand-grip 10 may include one or more detents for a user's fingers so improve the comfort thereof.

The smart exercise handle 100 is further provided with a ground-engaging section 20. The ground-engaging section 20 extends from the hand-grip 10. The ground-engaging section 20 is arranged to contact the ground so as to support the smart exercise handle 100 during exercise. For example, the ground-engaging section 20 may stably support the smart exercise handle 100 during bodyweight exercise such as push-ups.

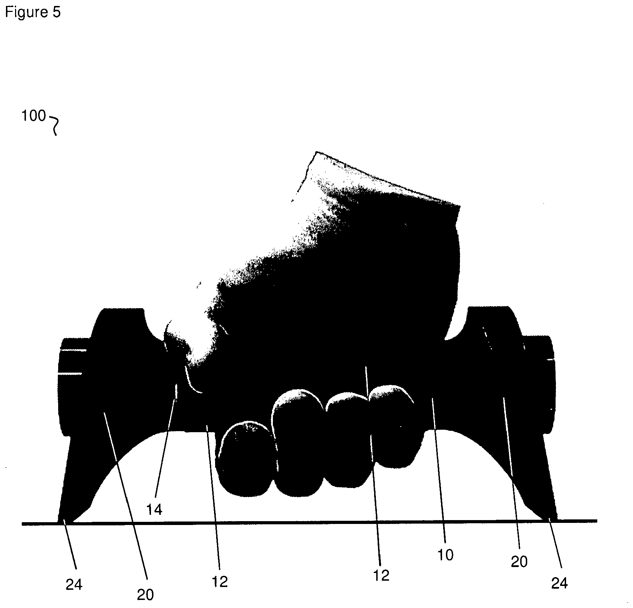

A user may grip the hand-grip 10 with one of their hands, with the ground-engaging section 20 contacting the floor. This is shown, for example, in Figure 5 with the user gripping the smart exercise handle 100 with their right hand. From this position, the user may perform push-ups. Typically, the user will grip a second exercise handle with their other hand. This second exercise handle may be a smart exercise handle 100 as described in the present description. Alternatively, the second exercise handle may be a simple exercise handle with no smart functionality. Of course, even if the second exercise handle is a smart exercise handle 100, certain smart functionality may be performed by only one of the smart exercise handles 100 and/or on a remote user device such as a smart phone.

The ground-engaging section 20 may be of any suitable design for supporting the smart exercise handle 100 during such exercise. For example, the hand-grip 10 may extend away from the ground-engaging section 20 such that the hand-grip 10 is generally cantilevered. In certain examples, such as shown in Figures 1 to 3, the ground-engaging section may comprise a first ground-engaging protrusion 24 and a second ground-engaging protrusion 24. The hand-grip 10 may extend between the first ground-engaging protrusion 24 and the second ground-engaging protrusion 24.

As shown in Figures 1 to 3, the first ground-engaging protrusion 24 may be angled with respect to the second ground-engaging protrusion 24. That is, the first ground-engaging protrusion 24 and the second ground-engaging protrusion 24 extend such that they are transverse to one another, or non-parallel. Particularly, the first ground-engaging protrusion 24 and the second ground-engaging protrusion 24 may be outwardly-splayed. That is, the first ground-engaging protrusion 24 and the second ground-engaging protrusion 24 may each extend from the hand-grip 10 away from the centre of the hand-grip 10. Geometrically, if a plane is defined which is orthogonal to the longitudinal axis of the hand-grip 10, the first ground-engaging protrusion 24 and the second ground-engaging protrusion 24 may be angled (i.e. transverse or non-parallel) with respect to that plane.

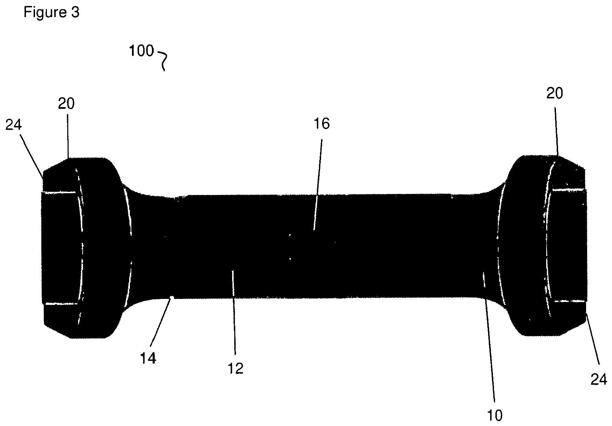

As can be seen in the top view of Figure 3, the ground-engaging section 20 may have a width greater than a width of the hand-grip 10. The width of the hand-grip 10 may be a diameter thereof. The first ground-engaging protrusion 24 and the second ground-engaging protrusion 24 may each have a width greater than a width of the hand-grip 10.

The smart exercise handle 100 further comprises at least one force sensor 12 arranged on the hand-grip 10. The at least one force sensor 12 is arranged to detect a force applied to the hand-grip 10. The at least one force sensor 12 then generates a signal indicative of the force applied to the hand-grip 10. The force applied to the hand-grip 10 is, during exercise, a force applied by the user to the hand-grip 10 and thus to the smart exercise handle 100. For example, during a push-up the user applies a force to the hand-grip 10 and through the smart exercise handle 100 as they straighten their arms during the pushing section of the push-up.

Specifically, the forces applied through the smart exercise handle 100 during a push up may include a component due to the weight of the user, which is generally constant. There is then a force applied in pushing up as the user straightens their arms which is added to the constant weight force. While the user bends their arms for a downward stroke in the push up there can be a relief force due to the drop in resistance which is subtracted from the constant weight force.

As discussed in more detail below, the smart exercise handle 100 may include one or more sensing areas, each arranged to detect a force applied to the hand-grip 10 in a different direction. The forces applied to multiple sensing areas may be resolved in order to determine a net direction of force applied to the smart exercise handle 100. This net direction of force can be used to determine details of exercise being carried out with the smart exercise handle 100.

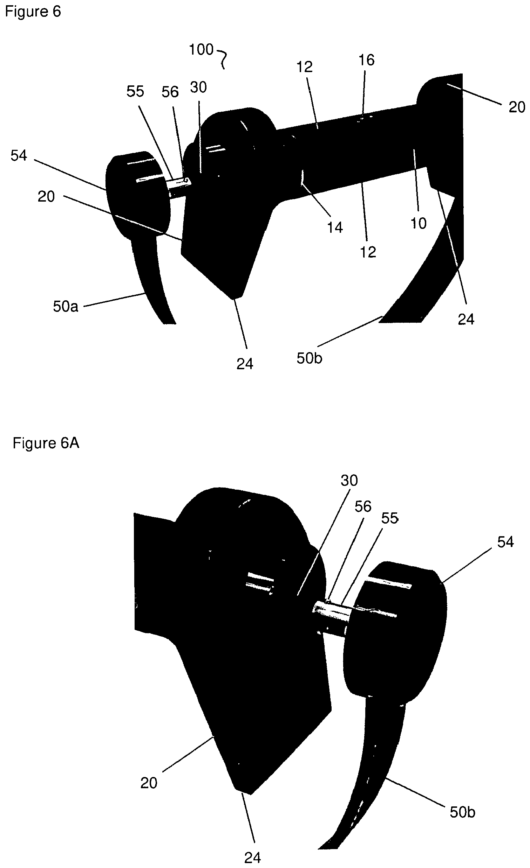

As will be discussed in more detail below in relation to Figures 6 to 8, the smart exercise handle 100 may further comprise an attachment section 30 for attaching a resistance band 50 to the smart exercise handle 100. When the user moves the smart exercise handle 100 against the resistive force of the resistance band 50 they apply a force to the hand-grip 10 and thus to the smart exercise handle 100.

The smart exercise handle 100 further comprises a processor. The processor can be any suitable component, including for example a microcontroller.

This processor is configured to receive the signal generated by the at least one force sensor 12. The processor then generates information related to the force applied to the hand-grip 10. This information could include statistics and/or analysis of the raw force signals. Alternatively, or additionally, the information could simply be the raw signal itself. The generated information may be presented to a user to provide them information about their workout. For example, this information may be presented via one or more of a visual display, an audible signal, and/or tactile feedback. The information may be a number of repetitions of an exercise carried out using the smart exercise handle 100. For example, if the exercise is push-ups each repetition may be a single push-up.

The smart exercise handle 100 may comprise a transmitter which is arranged to communicate with a remote receiver, such as a remote device. The transmitter may transmit one or both of the signal indicative of the force applied to the hand-grip 10 and/or the information generated by the processor related to the force applied to the hand-grip 10. The remote device may be, for example, a mobile phone or smart phone. Of course, the remote device could be any suitable device including a computer, tablet, wearable device, smart watch, or other device with a receiver. This transmitter may use any suitable communication protocol. For example, the transmitter may be configured to use Bluetooth and/or Wi-Fi.

In certain examples, a plurality of smart exercise handles 100 may communicate, via the transmitter, with a single remote device. For example, the single remote device may be a central server. This single remote device can then collate information from the smart exercise handles 100. The single remote device may co-ordinate an exercise regime across the plurality of smart exercise handles 100, such as in a gym class.

The smart exercise handle 100 may be generally hollow, with internal electronics. This may include, for example, any of the components discussed in the present specification.

In particular examples, the remote device may present information to the user related to the exercise they are performing with the smart exercise handle 100.

The smart exercise handle 100 may comprise further sensing components arranged to detect one or more characteristics of the exercise being performed with the smart exercise handle 100 and/or the user. For example, the smart exercise handle 100 may include one or more of: an accelerometer; and/or a gyroscope. Alternatively, or additionally, the smart exercise handle 100 may comprise a heart-rate sensor 16. The heart-rate sensor 16 may be arranged on any suitable part of the smart exercise handle 100, such as the hand-grip 10. These further sensing components, such as an accelerometer; and/or a gyroscope, may be used to determine a type of exercise being carried out. This determination may, for example, include data from the at least one force sensor 12. The further sensing components can also be used to determine one or more of progress in an exercise, a number of repetitions of elements of an exercise, the "form" of a user during an exercise, and/or a speed of a user during an exercise. Any combination of two or more of these further sensing components may be used in the smart exercise handle 100.

The smart exercise handle 100 may further comprise a vibration generator, such as a vibration motor. That is, a motor with an offset weight attached to its output shaft. The vibration generator may be provided in an interior of the smart exercise handle 100. Preferably, the vibration generator may be attached to a component forming an outer surface of the smart exercise handle 100, on the inner side of such a component.

The smart exercise handle 100 may further comprise a display means for communicating information. For example, this may be a screen. In other examples, this may include one or more LEDs 14. The one or more LEDs 14 can be used to communicate information related to the force applied to the hand-grip 10.

Examples of information related to the force applied to the hand-grip 10 which may be displayed to the user, such as via the one or more LEDs14 and/or via a remote device may include one or more of: heart rate; a number of exercise repetitions; calories burnt; position on a leader board; force applied; form correctness; progress of current repetition.

In this sense, the smart exercise handle 100 may be used for a number of different types of exercise and data relating to this exercise may be collected and presented to the user.

The at least one force sensor 12 of the smart exercise handle 100 can have various forms which are suitable for detecting a force applied to the hand-grip and generating a signal indicative thereof. In one example, the at least one force sensor may be formed as a force sensitive film 12. In the depicted embodiments there is an elastomeric padding layer 12A provided outwardly of the force sensitive film 12 (as discussed in more detail below). Thus, the force sensitive film 12 may not strictly be visible in the view of the Figures. Thus, the element identified as the force sensitive film 12 in the Figures may in fact be an elastomeric padding layer 12A.

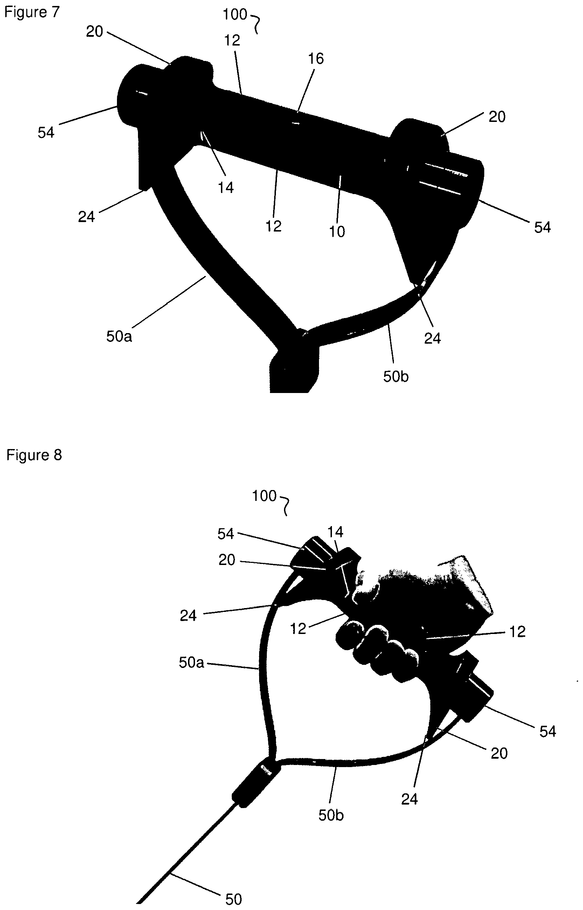

The force sensitive film 12 may have a thickness of 3 mm or less. In particular examples the force sensitive film 12 may have a thickness of 1 mm or less. An exemplary force sensitive film 12 is shown in Figure 8. The force sensitive film 12 may be a force sensitive resistor film 12.

This force sensitive film 12 may be wrapped around at least a portion of the hand-grip 10. That is, the force sensitive film 12 may be applied on an outer surface of the hand-grip 10. The hand-grip 10 may include a cavity formed on the outer surface of the hand-grip 10 for receiving this force sensitive film 12. This may mean that the outer surface of the hand-grip 10 adjacent the force sensitive film 12 and the force sensitive film 12 collectively form a generally continuous surface.

The force sensitive film 12 may be wrapped around a majority of the circumference of the hand-grip 10. That is, taking a cross-section of the hand-grip 10 in a direction orthogonal to the longitudinal axis, the force sensitive film 12 may overlie the cross-section over more than half of the circumference of the cross-section. In particular embodiments, the force sensitive film 12 may be wrapped around more than 80% of the circumference of the hand-grip 10. Preferably, this may be as high as 90% or more of the circumference of the hand-grip 10.

As can be seen in Figure 5, a section of the circumference which is not covered by the force sensitive film 12 may correspond to a region which is not held by a user during exercise such as push-ups. Alternatively, a section of the circumference which is not covered by the force sensitive film 12 may correspond to a region which is held by the user's hand. For example, this section may be provided at a region where the user's hand is wider than the uncovered area so that forces applied to the hand-grip 10 are still detected. A further gap in the force sensitive film may be provided to align with a heart rate sensor 16 such as shown in Figures 1 to 3.

In further examples, the force sensitive film 12 may be wrapped around generally the entire circumference of the hand-grip 10. This may leave a small gap where the edges of the force sensitive film 12 meet. For example, this may be more than 95% of the circumference.

The force sensitive film 12 may cover the hand-grip 10 to a longitudinal extent which may correspond to the width of an average user's hand. For example, the longitudinal extent of the force sensitive film 12 may be at least 70 millimetres.

The smart exercise handle 100 may further comprise an elastomeric padding layer 12A which is arranged adjacent to the force sensitive film 12. The elastomeric padding layer 12A may be formed of any suitable material, such as a foam padding, rubber or silicon. In preferred examples the elastomeric padding layer 12A may be silicon. The elastomeric padding layer 12A may have a thickness of 3 mm or less. In particular examples the elastomeric padding layer 12A may have a thickness of 1 mm or less. Of course, the thickness of the elastomeric padding layer 12A can be selected based on what is appropriate for a given smart exercise handle 100. This could be up to 10 mm or even greater. This elastomeric padding layer 12A may overlie the entire extent of the force sensitive film 12 to thereby cushion forces applies to the force sensitive film 12. That is, starting from the radial centre the smart exercise handle 100 may comprise the hand-grip 10, then the force sensitive film 12, and then the elastomeric padding layer 12A.

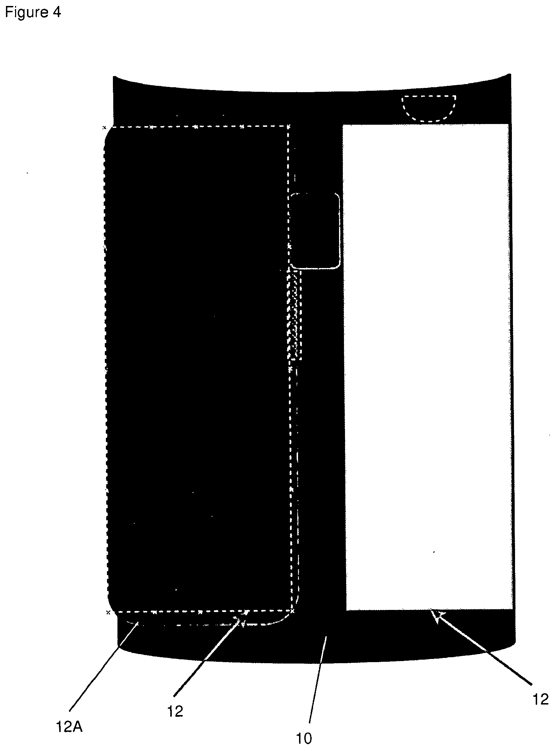

Figure 4 shows a section of the hand-grip 10. This section of hand-grip 10 has effectively been unwrapped onto a plane from its generally cylindrical shape. The left-hand side of Figure 4 includes an elastomeric padding layer 12A, while the right-hand side of Figure 4 does not. This is simply for ease of reference. The dotted line on the left-hand side of Figure 4 shows the extent of the force sensitive film 12 under the elastomeric padding layer 12A. As can be seen from Figure 4, the elastomeric padding layer 12A may extend beyond the outermost extent of the force sensitive film 12.

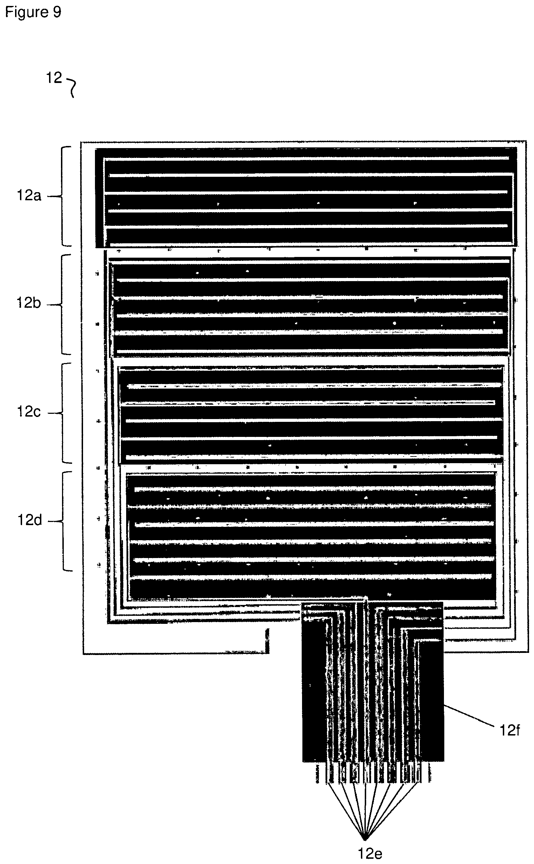

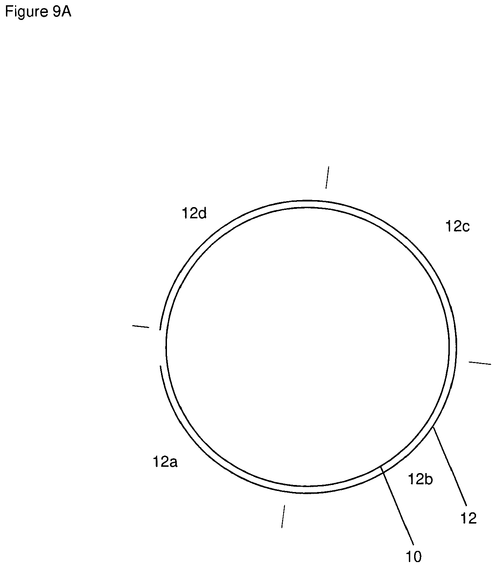

With particular reference to Figure 9, the force sensitive film 12 may be formed of a plurality of conductive tracks 12d. The force sensitive film 12 may be separated into a plurality of sensing areas 12a - 12d. Each sensing area 12a - 12d may be arranged to detect a force applied to the hand-grip 10 in different directions. That is, in a different direction to the direction of force applied which is sensed by the other sensing areas 12a - 12d. The force sensitive film 12 may be wrapped around the hand-grip 10 as shown in Figure 9A.

In particular examples, each sensing area 12a - 12d may have a corresponding sensing area 12a - 12d. With a sensing area 12a - 12d arranged to detect a force applied to the hand-grip 10 in a particular direction, the corresponding sensing area 12a - 12d is arranged to detect a force applied to the hand-grip 10 in an opposite direction. The direction of force detected by a sensing area 12a - 12d may be defined as a reference direction, and the corresponding sensing area detects a force applied in an opposite direction to the reference direction. In further examples, the directions may not be directly opposite but may instead simply be offset from one another.

For example, in Figures 9 and 9A the sensing film 12 is separated into four sensing areas 12a - 12d. Of course, there may be more or fewer sensing areas 12a - 12d. When wrapped around the hand-grip 10 the four sensing areas 12a - 12d effectively separate the hand-grip into four segments. Of course, the number of sensing areas 12a - 12d correspond to the number of segments. Each sensing area 12a - 12d has a corresponding sensing area 12a - 12d arranged on a generally opposite side of the hand-grip 10. For example, in Figure 9A the sensing area 12a has corresponding sensing area 12c. Of course, this means for sensing area 12c the corresponding sensing area is 12a. This is likewise true for sensing area 12b and sensing area 12d. in this sense, the sensing area 12a - 12d and its corresponding sensing area 12a - 12d may be defined as a pair.

In examples where the hand-grip 10 is generally circular in cross-section, each sensing area 12a - 12d and its corresponding sensing area 12a - 12d may be arranged at antipodal points of the cross-section. Of course, the sensing area 12a - 12d covers a region and not a point alone. Thus, it may just be that these antipodal points are encompassed within the sensing areas 12a - 12d. That is, a sensing area 12a - 12d and its corresponding sensing area 12a - 12d may be diametrically opposed. These sensing areas 12a - 12d and their corresponding sensing area are essentially on opposite sides of the hand-grip 10. As shown in Figure 9A, the force sensitive film 12 may not cover the entirety of every sensing area 12a - 12d. For example, there may be an uncovered portion of the hand-grip 10 as discussed above.

The force sensitive film 12 may generate a signal indicative of the force applied to the hand-grip 10 in the reference direction for each sensing area 12a - 12d. This can be referred to as the signal from a sensing area 12a- 12d. For example, as shown in Figure 9 there may be a plurality of conductive tracks corresponding to the plurality of sensing areas 12a - 12d. The force sensitive film 12 may comprise a protruding cable section 12f. This may terminate, for example, in any suitable connector. This cable section may pass through a corresponding slit formed in the smart exercise handle 100 to the internal electronics.

The processor may be configured to compare the signal from each sensing area 12a - 12d to the signal from the corresponding sensing area 12a - 12d. This comparison can be used to characterise and/or determine a type of exercise being carried out with the smart exercise handle 100. This may allow for a counter forces calculation to be carried out. That is, forces acting in a given axis (negative and positive) can be summed to determine the net force along that axis applied to the smart exercise handle 100.

This determination may also include data from further sensing components, such as an accelerometer; and/or a gyroscope, to determine the type of exercise. This determination could include determining that no exercise is being carried out - for example if someone is attempting to deceive the smart exercise handle 100. For example, is a user were to squeeze the handle there would be forces applied to both a sensing area 12a - 12d and its corresponding sensing area 12a - 12d. The processor could detect this and determine that no exercise is being performed. This would contrast to a push-up for example where the force applied to the hand-grip 10 would primarily be through one or more sensing areas 12a - 12d and not their corresponding sensing areas 12a - 12d.

The processor may further compare the signal from adjacent sensing areas 12a - 12d of the plurality of sensing areas 12a - 12d. This can be used, for example, to determine a net force applied to the smart exercise handle 100. It may be, for example, that a force is applied to the smart exercise handle 100 in a direction directly between two adjacent sensing areas 12a - 12d. As a result, there would be a component of this force in the adjacent sensing areas 12a - 12d. The comparison method discussed above can equally be applied to such a force based on the signal of multiple adjacent sensing areas 12a - 12d.

In alternative examples, the at least one force sensor 12 may be a plurality of force sensors 12 arranged around the hand-grip 10. For example, this may be a plurality of force sensitive resistors 12, but equally could be any other type of force sensor 12. In these examples, there may again be an elastomeric padding layer 12A provided over the at least one force sensor 12 in the same manner as described above in relation to a force sensitive film 12.

The plurality of force sensors 12 may be separated into a plurality of sensing areas 12a - 12d. These sensing areas 12a - 12d may be as described above in relation to the force sensitive film 12 unless otherwise specifically noted. Each sensing area 12a - 12d includes at least one force sensor 12 of the plurality of force sensors 12. The force sensor 12 of each sensing area 12a - 12d may be arranged to detect a force applied to the hand-grip 10 in a different direction to the force sensors 12 of the other sensing areas 12a - 12d.

In the same manner as described above in relation to the force sensitive film 7, each sensing area 12a - 12d may have a corresponding sensing area 12a - 12d. The sensing area 12a - 12d has a force sensor 12 arranged to detect a force applied to the hand-grip 10 in a first, reference direction. The corresponding sensing area 12a - 12d for that sensing area 12a - 12d then has a force sensor 12 arranged to detect a force applied to the hand-grip 10 in an opposite direction to the reference direction.

Again, there may be any number of sensing areas 12a - 12d as appropriate. Each sensing area 12a- 12d may have a corresponding signal indicative of force applied to the hand-grip 10 in the reference direction for that sensing area 12a - 12d. These signals may be transmitted to the processor such that the processor is configured to compare the signal from each sensing area 12a - 12d to the signal of the corresponding sensing area 12a - 12d. The processor may then operate as described above in relation to the force sensitive film 12.

The smart exercise handle 100 may be attachable to a resistance band 50. The resistance band 50 may be formed of any elastomeric material such that it provides a resistive force to being stretched. Figures 6 and 6A show the attachment of the resistance band 50 to the smart exercise handle 100, to form the assembly shown in Figure 7 which is shown in use for exercise in Figure 8.

The smart exercise handle 100 may comprise an attachment section 30 for removably attaching the resistance band 50 to the smart exercise handle 100. By removably attaching it is meant that the resistance band 50 can be attached to and detached from the smart exercise handle 100 repeatedly and/or non-destructively. The resistance band 50 may have a corresponding attachment mechanism 54 for engaging with the attachment section 30. This attaches the resistance band 50 to the smart exercise handle 100. In certain examples, the smart exercise handle 100 may comprise a plurality of attachment points 30 and the resistance band 50 may comprise a corresponding plurality of attachment mechanism 54. For example, the attachment section 30 may comprise a first attachment point 30 and a second attachment point 30. The resistance band 50 may then comprise a first attachment mechanism 54 and a second attachment mechanism 54.

In examples with first attachment point 30 and second attachment point 30, these may be at opposite ends of the hand-grip 10. That is, the hand-grip 10 may extend between the first attachment point 30 and the second attachment point 30. Each attachment point 30 may be arranged on a central longitudinal axis of the hand-grip 10. For example, each attachment point 30 may correspond to a bore formed in the hand-grip 10. If the hand-grip 10 is generally circular in cross-section this bore can be concentric with the circular cross-section. The attachment section 30 may allow for free rotation of the resistance band 50 when attached. That is, the resistance band 50 may be free to rotate 360° around the hand-grip 10.

The attachment mechanism 54 of the resistance band 50 may be any suitable mechanism. In specific examples, the attachment mechanism may comprise a ball lock pin. That is, the attachment mechanism 54 may comprise a pin 55 protruding therefrom. At a point along this pin 55, such as the distal end, there are one or more balls 56. These balls 56 are biased in a radially outward direction from the centre of the pin 55, such as via a spring. The pin 55 is then inserted into a bore hole of the attachment section 30. This bore may include an interior groove into which the balls 56 are forced by the bias so as to lock the resistance band 50 to the smart exercise handle 100.

To remove the resistance band 50 from the smart exercise handle 100 the pin 55 is pulled from the bore. This pulling must overcome the bias of the balls 56 to remain in the interior groove in order to remove the resistance band 50 from the smart exercise handle 100.

The resistance band 50 may have attachment mechanisms 54 at each end thereof. That is, the resistance band 50 may extend between a first end and a second end. Each of these ends may have one or more attachment mechanism 54. In particular examples, each end may have two attachment mechanisms 54. In this sense, there may be a first attachment mechanism 54 and a third attachment mechanism 54 at one end of the resistance band 50 and a second attachment mechanism and a fourth attachment mechanism 54 at the other end of the resistance band 50. These may each be a ball lock pin as described above, or any other attachment mechanism 54.

The resistance band 50 may comprise a central segment which then separates into strands 50a, 50b at each end. For example, the resistance band 50 may separate into two strands 50a, 50b at each end. In this sense, each strand 50a, 50b may comprise an attachment mechanism 54.

Multiple resistance bands 50 may be provided with different resistances to stretch and hence different force levels are required to stretch these resistance bands 50.

With the resistance band 50 attached, the user may grip the smart exercise handle 100 as shown in Figure 8 and move the smart exercise handle 100 against a resistive force provided by stretching the resistance band 50. For example, the resistance band 50 may be connected to two exercise handles (at least one of which is a smart exercise handle 100 as described herein) and the user may pull these exercise handles in opposite directions to thereby stretch the resistance band 50.

Collectively, one or more kits may be defined.

A first kit may comprise two exercise handles, at least one of which is a smart exercise handle 100 as described herein and a resistance band 50. Of course, both exercise handles may be smart exercise handles 100 as described herein. If the other exercise handle is not a smart exercise handle 100, it may still comprise an attachment section for attaching the resistance band 50.

Such a first kit may be used for exercises including resistance band work as well as bodyweight work such as push-ups.

This first kit may comprise a plurality of resistance bands 50, with different elastic modulus. A resistance band 50 with an appropriate elastic modulus and hence resistance to stretching may be selected based on the exercise to be performed.

A second kit may be defined with simply comprises two exercise handles, at least one of which is a smart exercise handle 100 as described herein without the resistance band 50. Of course, both exercise handles may be smart exercise handles 100.

Such a second kit may be used for bodyweight work such as push-ups.

The present disclosure also provides a number of methods.

A first method is a method of operating a smart exercise handle 100. This smart exercise handle 100 may be as described above. The method includes receiving, with the processor, data from the at least one force sensor 12. The processor then generates the information relating to the force applied to the hand-grip 10. This information may be any suitable information such as described above.

Finally this information is indicated to a user. The user may be a person using the smart exercise handle 100 to perform a workout. As discussed above, the information may be indicated to the user in any suitable format which may be via a display means on the smart exercise handle 100 such as LED 14. The smart exercise handle 100 could include a vibration generator for providing haptic feedback of the information. Alternatively, or additionally, this could be via a remote device such as a mobile phone.

A further method of operating a smart exercise device 100 is provided for a smart exercise device 100 which includes sensing zones 12a - 12d as discussed above. The method includes receiving, with the processor, a signal indicative of force applied to the hand-grip 10 in a reference direction from a first sensing area 12a of the plurality of sensing areas 12a - 12d. This signal is then compared, with the processor, to a signal indicative of force applied to the hand-grip 10 in an opposite direction to the reference direction. This opposite-direction signal is from a corresponding sensing area 12c of the plurality of sensing areas 12a - 12d. A type of exercise being performed is then determined or characterised.

The method may include receiving, with the processor, the signal indicative of force applied to the hand-grip 10 in an opposite direction to the reference direction from the corresponding sensing area 12c.

As noted above, the determination of the type of exercise may include determining that no exercise is being carried out. This may be, for example, is a user is attempting to deceive the smart exercise device 100.

In this sense, a smart exercise device 100 is provided that solves the problems associated with the prior art. It is small and easily transportable and thereby allows for effective use as a home workout device. A number of corresponding kits and methods are also provided.

### CLAUSES:

1. A smart exercise handle for bodyweight or resistance exercise, the handle comprising:
   a hand-grip for a user to grip;
   a ground-engaging section extending from the hand-grip, the ground-engaging section for contacting the ground to support the smart exercise handle during exercise;
   at least one force sensor arranged on the hand-grip to detect a force applied to the hand-grip and generate a signal indicative thereof;
   a processor configured to receive the signal and generate information related to the force applied to the hand-grip.
2. The smart exercise handle of clause 1, wherein the at least one force sensor is formed as a force sensitive film wrapped around at least a portion of the hand-grip, preferably the force sensitive film is wrapped around a majority of the circumference the hand-grip.
3. The smart exercise handle of clause 2, further comprising an elastomeric padding layer covering the force sensitive film,
   preferably the elastomeric padding layer is silicon.
4. The smart exercise handle of clause 2 or 3, wherein the force sensitive film is separated into a plurality of sensing areas, each sensing area arranged to detect a force applied to the hand-grip in a different direction.
5. The smart exercise handle of clause 4, wherein each sensing area is arranged to detect a force applied to the hand-grip in a reference direction for that sensing area, and there is a corresponding sensing area arranged to detect a force applied to the hand-grip in an opposite direction to the reference direction,
   wherein the processor is configured to compare the signal from each sensing area to the signal from the corresponding sensing area.
6. The smart exercise handle of clause 1, wherein the at least one force sensor comprises a plurality of force sensitive resistors separated into a plurality of sensing areas, each sensing area comprising at least one force sensitive resistor of the plurality of force sensitive resistors and arranged to detect a force applied to the hand-grip in a different direction.
7. The smart exercise handle of clause 6, the at least one force sensitive resistor in each sensing area being arranged to detect a force applied to the hand-grip in a reference direction for that sensing area, and the at least one force sensitive resistor in a corresponding sensing area being arranged to detect a force applied to the hand-grip in an opposite direction to the reference direction,
   wherein the processor is configured to compare the signal from each sensing area to the signal from the corresponding sensing area.
8. The smart exercise handle of any of clauses 4 to 7, wherein the plurality of sensing areas is four sensing areas.
9. The smart exercise handle of any of clauses 4 to 8, wherein the hand-grip is generally circular in cross-section and each sensing area and its corresponding sensing area are diametrically opposed in the cross-section.
10. The smart exercise handle of any preceding clause, wherein the information related to the force applied to the hand-grip is a number of repetitions of an exercise carried out using the smart exercise handle.
11. The smart exercise handle of any preceding clause, further comprising, connected to the processor, one of more of:
   an accelerometer;
   a gyroscope; and/or
   a heart-rate sensor.
12. The smart exercise handle of any preceding clause, further comprising a vibration generator.
13. The smart exercise handle of any preceding clause, further comprising a transmitter arranged to transmit the signal indicative of a force applied to the hand-grip and/or the information related to the force applied to the hand-grip to a remote device,
   preferably the remote device is a mobile phone.
14. The smart exercise handle of any preceding clause, wherein the ground-engaging section comprises a first ground-engaging protrusion and a second ground-engaging protrusion, with the hand-grip extending therebetween.
15. The smart exercise handle of clause 14, wherein the first ground-engaging protrusion is angled with respect to the second ground-engaging protrusion such that first ground-engaging protrusion and the second ground-engaging protrusion are outwardly-splayed.
16. The smart exercise handle of clause 14 or 15, wherein the first ground-engaging protrusion and the second ground-engaging protrusion each have a width greater than the width of the hand-grip.
17. The smart exercise handle of any preceding clause, further comprising an attachment section for attaching a resistance band to the smart exercise handle.
18. The smart exercise handle of clause 17, wherein the attachment section comprises a first attachment point and a second attachment point, with the hand-grip extending therebetween.
19. The smart exercise handle of clause 18, wherein the hand-grip defines a central longitudinal axis extending along the hand-grip, and the first attachment point and the second attachment point are positioned on the central longitudinal axis.
20. The smart exercise handle of any of clauses 17 to 19, wherein the attachment section is configured to allow free rotation of the resistance band.
21. A kit comprising:
   a first exercise handle according to any of clauses 17 to 20;
   a second exercise handle comprising an attachment section for attaching a resistance band; and
   a first resistance band comprising a first attachment mechanism at a first end and a second attachment mechanism at a second opposite end for attaching to the attachment sections of the first exercise handle and the second exercise handle.
22. The kit of clause 21, wherein the first attachment mechanism and the second attachment mechanism each comprise a ball lock pin for engaging with the attachment sections of the first exercise handle and the second exercise handle.
23. The kit of clause 21 or 22, wherein the first resistance band further comprises a third attachment mechanism at the first end and a fourth attachment mechanism at the second opposite end.
24. The kit of clause 23 where the first resistance band comprises a central segment which splits into two strands at the first and the second end, with each strand having an attachment mechanism.
25. The kit of any of clauses 21 to 24, wherein the second exercise handle is according to any of clauses 17 to 20.
26. A kit comprising:
   a first exercise handle and a second exercise handle, wherein:
   the first exercise handle is according to any of clauses 1 to 20; and/or
   the second exercise handle is according to any of clauses 1 to 20.
27. A method of operating a smart exercise handle according to any of clauses 1 to 20, comprising the steps of:
   receiving, with the processor, a signal from the at least one force sensor;
   generating, with the processor, information relating to the force applied to the hand-grip; and
   indicating the information to a user.
28. A method of operating a smart exercise handle according to clause 5 or 7, comprising the steps of:
   receiving, with the processor, a signal indicative of force applied to the hand-grip in a reference direction from a first sensing area of the plurality of sensing areas;
   comparing, with the processor, the received signal to a signal indicative of force applied to the hand-grip in an opposite direction to the reference direction from a corresponding sensing area of the plurality of sensing areas to determine a type of exercise being carried out.

## Claims

1. A smart exercise handle for bodyweight or resistance exercise, the handle comprising:
a hand-grip for a user to grip;
a ground-engaging section extending from the hand-grip, the ground-engaging section for contacting the ground to support the smart exercise handle during exercise;
at least one force sensor arranged on the hand-grip to detect a force applied to the hand-grip and generate a signal indicative thereof;
a processor configured to receive the signal and generate information related to the force applied to the hand-grip.

2. The smart exercise handle of claim 1, wherein the at least one force sensor is formed as a force sensitive film wrapped around at least a portion of the hand-grip, preferably the force sensitive film is wrapped around a majority of the circumference the hand-grip,
preferably the smart exercise handle, further comprises an elastomeric padding layer covering the force sensitive film.

3. The smart exercise handle of claim 2, wherein the force sensitive film is separated into a plurality of sensing areas, each sensing area arranged to detect a force applied to the hand-grip in a different direction.

4. The smart exercise handle of claim 3, wherein each sensing area is arranged to detect a force applied to the hand-grip in a reference direction for that sensing area, and there is a corresponding sensing area arranged to detect a force applied to the hand-grip in an opposite direction to the reference direction,
wherein the processor is configured to compare the signal from each sensing area to the signal from the corresponding sensing area.

5. The smart exercise handle of claim 1, wherein the at least one force sensor comprises a plurality of force sensitive resistors separated into a plurality of sensing areas, each sensing area comprising at least one force sensitive resistor of the plurality of force sensitive resistors and arranged to detect a force applied to the hand-grip in a different direction.

6. The smart exercise handle of claim 5, the at least one force sensitive resistor in each sensing area being arranged to detect a force applied to the hand-grip in a reference direction for that sensing area, and the at least one force sensitive resistor in a corresponding sensing area being arranged to detect a force applied to the hand-grip in an opposite direction to the reference direction,
wherein the processor is configured to compare the signal from each sensing area to the signal from the corresponding sensing area.

7. The smart exercise handle of any preceding claim, wherein the information related to the force applied to the hand-grip is a number of repetitions of an exercise carried out using the smart exercise handle.

8. The smart exercise handle of any preceding claim, further comprising one or more of:
an accelerometer;
a gyroscope;
a heart-rate sensor; and/or
a transmitter arranged to transmit the signal indicative of a force applied to the hand-grip and/or the information related to the force applied to the hand-grip to a remote device.

9. The smart exercise handle of any preceding claim, wherein the ground-engaging section comprises a first ground-engaging protrusion and a second ground-engaging protrusion, with the hand-grip extending therebetween.

10. The smart exercise handle of any preceding claim, further comprising an attachment section for removably attaching a resistance band to the smart exercise handle, preferably the attachment section comprises a first attachment point and a second attachment point, with the hand-grip extending therebetween,
more preferably the hand-grip defines a central longitudinal axis extending along the hand-grip, and the first attachment point and the second attachment point are positioned on the central longitudinal axis.

11. The smart exercise handle of claim 10, wherein the attachment section is configured to allow free rotation of the resistance band.

12. A kit comprising:
a first exercise handle according to any of claims 10 to 11;
a second exercise handle comprising an attachment section for attaching a resistance band; and
a first resistance band comprising a first attachment mechanism at a first end and a second attachment mechanism at a second opposite end for attaching to the attachment sections of the first exercise handle and the second exercise handle,
preferably the first attachment mechanism and the second attachment mechanism each comprise a ball lock pin for engaging with the attachment sections of the first exercise handle and the second exercise handle.

13. A kit comprising:
a first exercise handle and a second exercise handle, wherein:
the first exercise handle is according to any of claims 1 to 11; and/or
the second exercise handle is according to any of claims 1 to 11.

14. A method of operating a smart exercise handle according to any of claims 1 to 11, comprising the steps of:
receiving, with the processor, a signal from the at least one force sensor;
generating, with the processor, information relating to the force applied to the hand-grip; and
indicating the information to a user.

15. A method of operating a smart exercise handle according to claim 4 or 6, comprising the steps of:
receiving, with the processor, a signal indicative of force applied to the hand-grip in a reference direction from a first sensing area of the plurality of sensing areas;
comparing, with the processor, the received signal to a signal indicative of force applied to the hand-grip in an opposite direction to the reference direction from a corresponding sensing area of the plurality of sensing areas to determine a type of exercise being carried out.
